# EUROPEAN PATENT APPLICATION

(11) **EP 4 825 330 A1**
(43) Date of publication of application: **21.10.2026**
(21) Application number: 26171171.7
(22) Date of filing: 08.04.2026
(51) Int. Cl.: A61B 18/02

(54) **CRYOPROBE WITH FLEXIBLE HANDLE**

(30) Priority: 17.04.2025 US 202519181543
(71) Applicant: Varian Medical Systems, Inc., Palo Alto, CA 94304 (US)
(72) Inventor: GAUD, Ishan, Austin, 78717 (US)
(74) Representative: Mathisen & Macara LLP

(57) **Abstract**

A cryoprobe (112) includes a needle (114), and a handle (116, 120) configured to fluidly couple the needle to a cryogen line (110). The handle is configured to flex to change a position of the needle relative to the cryogen line.

## Description

### FIELD

The present disclosure relates to cryoablation probes that include flexible and/or adjustable handles.

### BACKGROUND

This section provides background information related to the present disclosure which is not necessarily prior art.

Systems and methods for providing cryoablation treatments may include cryoablation probes that are introduced at or near target tissue in a patient. A cryoablation system may include an extremely cold cryogen (liquid, gas, or mixed phase) that may be passed through a probe in thermal contact with the target tissue. Heat from the tissue passes from the tissue, through the probe, and into the cryogen that removes heat from the targeted tissue. This removal of heat causes tissue to freeze, resulting in the destruction of the targeted tissue. It is desirable that the cryogen is of sufficiently low temperature to quickly and efficiently cause the targeted tissues to freeze.

Traditional or existing cryoprobes and related structures often include rigid configurations that do not allow for flexing and/or adjustment at the handle portion of the cryoprobe. Such rigid configurations limit the ability to position the cryoprobe or may induce movement of the cryoprobe after it is initially positioned at a desired location for treatment. There exists a need, therefore, for cryoprobes that may have adjustable configurations such as the ability to flex at or near a handle portion of the cryoprobe. There is also a need to improve existing cryoprobes to prevent or limit movement of the cryoprobe during a cryoablation treatment.

### SUMMARY

This section provides a general summary of the disclosure, and is not a comprehensive disclosure of its full scope or all of its features.

In various embodiments of the present disclosure, a cryoablation probe is provided. The cryoablation probe may include a needle and a handle configured to fluidly couple the needle to a cryogen supply line. The handle may be configured to flex to change a position of the needle relative to the cryogen supply line.

In one aspect, the handle may be configured to flex from a first position in which a longitudinal axis of the needle is aligned with a longitudinal axis of the cryogen supply line to a second position in which the longitudinal axis of the needle is positioned at an angle relative to the longitudinal axis of the cryogen supply line.

In another aspect, the angle of the second position of the cryoprobe may be in a range between 180 degrees and 90 degrees.

In another aspect, the handle may include a deformable member configured to maintain a plurality of positions of the needle relative to the cryogen supply line. In an embodiment, the handle includes an outer member, a deformable member, and a cryogen supply tube, which may be positioned concentrically about a center axis of the handle.

In another aspect, the deformable member may be positioned radially inward and spaced apart from the outer member. In another aspect, the handle may include: a flexible outer member defining an inner cavity, and a deformable inner member positioned in the inner cavity, the deformable inner member configured to maintain a position of the needle relative to the cryogen supply line.

In another aspect, the flexible outer member and the deformable inner member each may have a cylindrical shape.

In another aspect, the handle may include a cryogen supply tube positioned radially inward of the deformable inner member in the inner cavity.

In another aspect, the deformable inner member and the cryogen supply tube may define a cryogen flow path in which a cryogen flows toward a tip of the needle through the cryogen supply tube and away from the tip of the needle in a space between an outer surface of the cryogen supply tube and an inner surface of the deformable inner member.

In another aspect, the handle may include a limiting member positioned in the inner cavity radially outward of the deformable inner member. For example, the limiting member may be positioned radially inward of the outer member and radially outward of the deformable member. The limiting member may be configured to limit bending of the handle. For example, the limiting member may limit an angle that the handle can be flexed. The limiting member may limit the angle of flex of the handle to about 90 degrees, in some examples. The limiting member may have a helical configuration. In other examples, the limiting member may have a coil spring configuration. In other examples, a corrugated or oscillating outer surface may be used to provide a limiting member. The limiting member may be a separate member and may be positioned in a space between concentric members of the handle. In other examples, the limiting member may integrally formed and/or embedded into one of the outer member and/or the deformable member.

In another aspect, the handle may include a cryogen supply tube and an outer flexible member. The outer flexible member may include at least one embedded wire configured to maintain a position of the needle relative to the cryogen supply line. The wire suitably provides a deformable member. The wire is suitably malleable.

In another aspect, the at least one embedded wire may include a plurality of embedded wires. Each of the wires may have the same cross-sectional diameter. In other examples, the wires may have different cross-sectional shapes, sizes, or diameters. In some examples, the wires may be positioned and/or sized to limit bending or flexing in a particular direction. For example, a wire having a larger diameter than another wire may be positioned on a side of the handle in which it is desired to limit bending in that particular direction.

In another aspect, each wire of the plurality of embedded wires may be equally spaced around a circumference of the outer flexible member.

In some embodiments, the handle of the cryoprobe may include a flexible outer member, and a deformable member configured to maintain a position of the needle relative to the cryogen supply line.

In another aspect, the flexible outer member may include at least one longitudinal reinforcement configured to limit bending of the flexible outer member in at least one plane.

In another aspect, the at least one longitudinal reinforcement may include a first reinforcement and a second reinforcement positioned on opposite sides of the flexible outer member. In other examples, other quantities or other orientations or positioning of the reinforcements may be used. The first reinforcement and the second reinforcement may include enlarged portions of a wall of the flexible outer member. The reinforcements may be projections that extend outward from an outer surface of the outer member. The reinforcements may be longitudinally formed in the outer member and may extend along the length of the outer member. The reinforcements may be integrally formed into the wall of the outer member. In other examples, the reinforcements may be separate pieces joined to the outer member or extruded with or into the material of the outer member. The reinforcements may have any suitable cross-sectional shape, such as a rectangular, triangular or circular shape.

In another aspect, the handle may be configured to flex in a plurality of planes relative to an axis of the cryogen supply line.

In another aspect, the handle may be configured to flex in at least two orthogonal planes relative to an axis of the cryogen supply line.

In another aspect, the handle may include a deformable member configured to permanently deform to maintain a position of the needle relative to the cryogen supply line.

In some embodiments, a cryoablation system is provided. The cryoablation system may include a cryoprobe of present disclosure and a cryoablation delivery apparatus configured to deliver a cryogen to the cryoprobe.

The method of using a cryoprobe may include: positioning a cryoprobe of the present disclosure at a target tissue, bending a handle of the cryoprobe from a first position to a second position to orient the cryogen supply line at a desired position relative to the needle, and initiating a flow of cryogen to the cryoprobe while the handle is in the second position.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
FIG. 1 is an illustration of an example cryoablation apparatus in accordance with some embodiments of the present disclosure.
FIG. 2 is a side cross-sectional view of an example cryoprobe in accordance with some embodiments of the present disclosure.
FIG. 3 is a cross-sectional view of an example flexible handle in accordance with some embodiments of the present disclosure.
FIG. 4 is a cross-sectional view of another example flexible handle in accordance with some embodiments of the present disclosure.
FIG. 5 is a cross-sectional view of another example flexible handle in accordance with some embodiments of the present disclosure.
FIG. 6 is a cross-sectional view of another example flexible handle in accordance with some embodiments of the present disclosure.

Corresponding reference numerals indicate corresponding parts throughout the several views of the drawings.

### DETAILED DESCRIPTION

Example embodiments will now be described more fully with reference to the accompanying drawings.

Example embodiments are provided so that this disclosure will be thorough, and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

The terminology used herein is for the purpose of describing particular example embodiments only and is not intended to be limiting. As used herein, the singular forms "a," "an," and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

When an element or layer is referred to as being "on," "engaged to," "connected to," or "coupled to" another element or layer, it may be directly on, engaged, connected or coupled to the other element or layer, or intervening elements or layers may be present. In contrast, when an element is referred to as being "directly on," "directly engaged to," "directly connected to," or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the example embodiments.

Spatially relative terms, such as "inner," "outer," "beneath," "below," "lower," "above," "upper," and the like, may be used herein for ease of description to describe one element or feature's relationship to another element(s) or feature(s) as illustrated in the figures. Spatially relative terms may be intended to encompass different orientations of the device in use or operation in addition to the orientation depicted in the figures. For example, if the device in the figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

In some embodiments of the present disclosure, a cryoprobe is provided that includes a flexible handle. Cryoprobes may include a handle that couples a needle of the cryoprobe to a cryogen line. The cryogen line may include one or more tubes, lumens or other conduits through which a cryogen (e.g., argon, nitrogen, etc.) may flow toward or away from the needle during operation of the cryoprobe. The cryogen line may include other elements as well such as wires for sending or receiving signals for control or measurement of aspects of the cryoprobe.

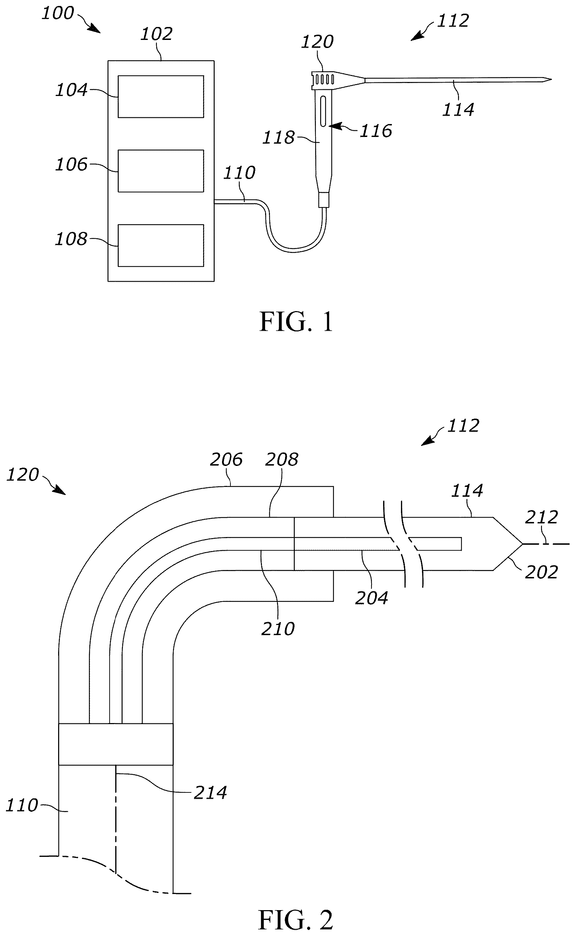

The handle of typical cryoprobes are often rigid in that they fix a relationship between the cryogen line and the needle of the cryoprobe. It should be understood that the cryogen line may be a length of flexible tubing that can change shape. In the present disclosure, when discussion is made of a relative relationship between the needle of the cryoprobe and the cryogen line, the relationship is defined between a longitudinal axis of the needle and a longitudinal axis of the cryogen line if the cryogen line were positioned in a linear manner extending from its connection to the handle of the cryoprobe. With reference to FIG. 2, the relationship between the cryogen line 110 and the needle 114 is discussed as a relationship between needle axis 212 and cryogen supply line axis 214. The cryogen supply line axis 214 is defined at the point of connection of the cryogen line 110 to the handle 120. As can be appreciated, the cryogen line 110 may bend as it travels to its connection to a cryogen apparatus (as shown in FIG. 1) but the axis 214 is defined, for purposes of the present disclosure, at the connection of cryogen line 110 to handle 120.

In various examples of existing cryoprobes, a handle of the cryoprobe may orient the needle in a linear relationship. In other examples, the handle of existing cryoprobes may fix the relationship between the needle and the cryogen line. Such angles may be 90 degrees, for example, in a right angle configured handle. It may be desirable to have flexibility and/or to have the ability to change a relationship between the needle and the cryogen line. For example, after the needle of a cryoablation probe is positioned at a target tissue in a patient, the patient may be moved in the treatment room for imaging or other procedures. During imaging, the patient may be inserted into an imaging apparatus in which there is limited space surrounding the patient. It may be desirable to change, bend or flex, the orientation of the cryogen line relative to the needle to allow for clearance around the patient for imaging or other supplemental procedures.

Still further, more than one cryoprobe may inserted into the patient during treatment. Such multiple probes are often inserted at positions very close to each other. The cryogen lines of the multiple probes may interfere with each other in such circumstances. It is desirable to be able to move the cryogen supply lines into positions away from each other to minimize or reduce interference with each other.

The functionality previously described is also desirable so that the needles of the cryoprobes remain in position once they are positioned in a desired location at or near the target tissue. If the cryogen supply lines touch or otherwise interfere with each other, it is possible that the needle of the cryoprobe may be moved impacting the effectiveness of the treatment. Also, it can be desirable to orient the cryogen lines in a manner to minimize movement of the needle.

The cryoprobes of the present disclosure include flexible handles that allow a user to move and/or position the cryogen lines at desired orientations relative to the needles of the cryoprobe. The users of the cryoprobes may manipulate the flexible handles of the present disclosure to position the cryogen lines so as to minimize or reduce interference with each or with other instruments or devices that may be used in a treatment room. Once a user bends or moves the handle of the cryoprobes of the present disclosure, the handle deforms in a manner to maintain the desired position of the handle. This assists in keeping the needle of the cryoprobe in its desired location relative to the target tissue to improve effectiveness of treatment.

Referring now to FIG. 1, an example cryoablation apparatus 100 is shown. The cryoablation apparatus 100 may include a cryoablation console 102, and a cryoprobe 112. The cryoablation console 102 may include a cryo-controller 104, a cryogen delivery apparatus 106 and a cryogen source 108. While shown inside console 102 in FIG. 1, the cryogen source 108 may be located outside the console 102. The cryo-controller 104 may include a computing device or other controller that can be used to control delivery of a cryogen (e.g., Argon, Helium, Nitrogen, or the like) from the cryogen source 108 to the cryoprobe 112 using the cryogen delivery apparatus 106. The cryogen source 108 may be a suitable Dewar or other container that can be filled with a cryogen. The cryogen delivery apparatus 106 may include a pump, one or more valves, and other suitable fluid delivery devices to fluidly connect the cryogen source to the cryogen line 110 of the cryoprobe 112. Upon the initiation of a freezing cycle, the cryo-controller 104 may cause the cryogen to be moved through a cryogen flow path that includes a cryogen supply line from the cryogen source through the cryogen line 110 to the cryoprobe 112. The cryogen may then flow back to the cryogen source via a cryogen return line from the cryoprobe 112 back to the cryogen source 108 or it may be vented to ambient air.

The cryogen line 110 may be a flexible tube or other conduit that may include multiple lumens to allow the cryogen to flow in a supply direction to the needle 114 and separately in a return direction away from the needle 114. The cryogen line 110 may of sufficient length to allow the console 102 to be positioned near the patient in a treatment room and to allow the patient to be moved into and out of an imaging device. In some examples, the cryogen line may be about 12 feet (3.66m) in length. In other examples, the cryogen line 110 may have other lengths. In other examples, the cryogen line 110 may be greater than about 12 feet (3.66m). In other examples, the cryogen line 110 may be less than about 12 feet (3.66m) in length.

The cryogen line 110 fluidly couples the cryogen source 108 to the cryoprobe 112. The cryoprobe 112 may include a needle 114. The needle 114 may be a pointed cylindrical tool or other elongated member that is configured to be inserted into patient tissue and be positioned at or near the target tissue during treatment. The needle 114 may be configured as a pointed tool having an outer diameter in a range of about 1 mm to about 4 mm. The cryoprobe 112 may also include a handle 116. The handle 116 may be flexed or moved to allow an orientation of the needle 114 and the cryogen line 110 to be changed or modified as desired.

In some examples, the handle 116 may include a vacuum chamber positioned at or near an outside surface of the handle 116. The vacuum chamber may insulate the exterior of the handle from the extremely low operating temperatures of the cryogen that moves through the handle to the cryoprobe 112. This may allow an operator to touch or otherwise manipulate the cryoprobe 112 during treatment.

While not shown, more than one cryoprobe 112 may be coupled to the console 102. Multiple cryoprobes 112 may be used during a single cryoablation treatment in combination. The console 102 may be configured to deliver cryogen to each of the multiple cryoprobes 112. The cryoprobes 112 may be similar to each other or may be different to produce iceballs of different sizes and shapes so as to freeze and destroy the target tissue.

Referring now to FIG. 2, an example cryoprobe 112 is shown. The cryoprobe 112 may be used in combination with a cryoablation apparatus, such as the cryoablation apparatus 100 previously described. In this example, the cryoprobe 112 may include a needle 114 and a handle 120. The cryoprobe 112 may be coupled to a cryogen source using a cryogen line 110. The needle 114 may be any suitable cryoablation needle and may extend in an axial direction away from handle 120 along axis 212. The needle 114 may be made of suitable metal or alloy such as stainless steel. In other examples, other suitable materials such as ceramic may be used. The needle 114 may have various sizes or diameters that may be suitable to produce a freezing zone or iceball that may be desired to freeze the target tissue. In various examples, the needle 114 may have an outer diameter in a range of about 1 mm to about 4 mm. In other examples, other suitable sizes may be used.

The needle 114 may include an outer shell and an inner lumen 204 that extends radially along its length. The outer shell and the inner lumen 204 may be positioned concentrically with each other. The inner lumen 204 may include an opening at one end toward a tip 202 of the needle. During a freezing cycle, the cryogen (at high pressure) may be passed through the inner lumen 204 toward the tip 202 and the cryogen may exit the inner lumen 204 and allowed to expand in a Joule-Thompson chamber at or near the tip 202. Such expansion causes the temperature of the cryogen to drop. The cryogen may then flow away from the tip 202 through a return pathway defined as a space between an inner surface of the outer shell and the outer surface of the inner lumen 204.

The needle 114 may be coupled to the handle 120. The handle 120 may be formed of plastic, composite, or other suitable flexible materials. The handle 120 may be fluidly and mechanically coupled to the needle 114. The inner lumen 204 and the outer shell of the needle 114 may be fluidly coupled to a supply lumen 210 and a return pathway that is positioned radially outward of the supply lumen 210, respectively. In this manner, the supply cryogen may be supplied to the needle 114 through the handle 120 and the return cryogen may flow from the needle 114 to the cryogen line 110.

The flexible handle 120 may include an outer member 206, a deformable member 208, and a cryogen supply tube 210. The outer member 206 may form the outer surface of the handle and may be formed of a flexible plastic tubing. The outer member may have wall thickness and a hollow interior that forms an inner cavity. The deformable member 208 may be positioned inside the inner cavity of the outer member 206. The deformable member 208 may be a malleable member that can be bent or formed into a desired shape and can maintain the deformed shape without external forces. The deformable member 208 may undergo plastic deformation and maintain a deformed shape. In some examples, the deformable member 208 may be formed of a brass or stainless steel tubing in an annealed state to allow the deformable member to be shaped into a desired shape without kinking or rupture.

The cryogen supply tube 210 may be positioned radially inward of the deformable member 208. The cryogen supply tube 210 may also be deformable but of a smaller outer diameter than the deformable member 208.

The handle 120 is flexible in that it can be shaped to position the needle 114 at various desired relative orientations relative to the cryogen line 110. The relative orientation of the needle 114 to the cryogen line 110 can be characterized by a relative position of the needle axis 212 to the cryogen line axis 214. As described above, the cryogen line axis 214 is defined at the connection plane of the cryogen line 110 to the handle 120. In various examples, the handle 120 is formed of suitable materials and wall thicknesses to allow the handle 120 to be moved from a first position to a second position to change an angle of the needle 114 (or needle axis 212) relative to the cryogen line 110 (or cryogen line axis 214). In some examples, the handle 120 may permit the angle of the needle 114 relative to the cryogen line 110 to be in a range of about 180 degrees to about 90 degrees. In some examples, the handle 120 may permit the angle of the needle 114 relative to the cryogen line 110 to be in a range of about 180 degrees to about 45 degrees. While FIG. 2 shows the flexible handle 120 in a single plane, the flexible handle 120 may be moved, flexed or bent in multiple planes such as planes normal to the view shown in FIG. 2 and at various planes between the orthogonal planes.

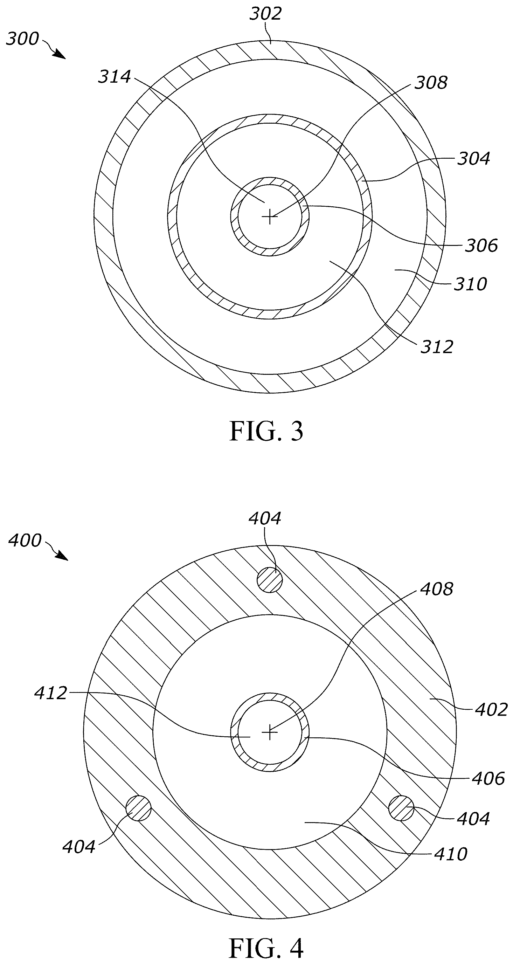

Referring now to FIG. 3, an example cross-section of a flexible handle 300 is shown. The flexible handle 300 may be similar to the handle 120 previously described in that it couples the needle 114 to the cryogen line 110 and permits relative movement between the needle 114 and the cryogen line 110. The flexible handle 300 may permit movement and positioning of the needle 114 relative to the cryogen line 110 as previously described.

In this example, the handle 300 includes an outer member 302, a deformable member 304, and a cryogen supply tube 306. The outer member 302, the deformable member 304, and the cryogen supply tube 306 may be made of the materials and have the relative positioning as previously described in the example shown in FIG. 2. The outer member 302, the deformable member 304, and the cryogen supply tube 306 may be positioned concentrically about a center axis 308 of the handle 300. The deformable member 304 may be positioned radially inward and spaced apart from the outer member 302. This positioning may define an annular space 310 between in inner surface of the outer member 302 and an outer surface of the deformable member 304. Similarly, the cryogen tube 306 may be positioned radially inward and spaced apart from the deformable member 304 to define an annular space 312. The interior of the cryogen tube 306 may define a cryogen lumen 314.

During use of the cryoprobe, the cryogen may flow through the cryogen tube 306 in the lumen 314 toward a tip of the needle. The cryogen may flow away from the needle through the annular space 312 between an outer surface of the cryogen tube 306 and the inner surface of the deformable member 304. The annular space 310 may be an air gap between the outer member 302 and the deformable member 304.

Referring now to FIG. 4, another example cross-section of a flexible handle 400 is shown. The flexible handle 400 may be similar to the handle 120 previously described in that it couples the needle 114 to the cryogen line 110 and permits relative movement between the needle 114 and the cryogen line 110. The flexible handle 400 may permit movement and positioning of the needle 114 relative to the cryogen line 110 as previously described.

In this example, the handle 400 includes an outer member 402, deformable members 404, and a cryogen supply tube 406. In this example, the deformable members 404 may be malleable wires positioned in a wall of the outer member 402 and centered around center axis 408. In this example, the handle 400 includes three deformable members 404 equally spaced at intervals around a circumference of the outer member 402. The deformable members 404 may be of suitable thickness to maintain a shape of the outer member when the handle 400 is shaped or bent into a desired configuration. While three deformable members 404 are shown in this example, other quantities of deformable members 404 may be used. In some examples, the handle 400 may include more than three deformable members 404. In other examples, the handle 400 may include less than three deformable members 404 or may include one deformable member 404.

In this example, each of the deformable members 404 have the same cross-sectional diameter. In other examples, the deformable members 404 may have different cross-sectional shapes, sizes, or diameters. In some examples, the deformable members 404 may be positioned and/or sized to limit bending or flexing in a particular direction. For example, a deformable member 404 having a larger diameter than another deformable 404 may be positioned on a side of the handle 400 in which it is desired to limit bending in that particular direction. In still other examples, other variations can be used.

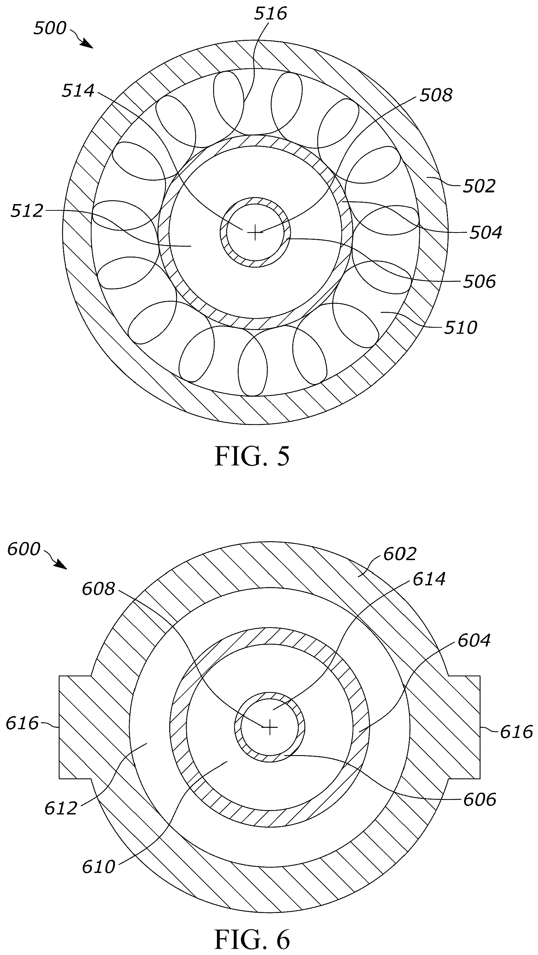

Referring now to FIG. 5, an example cross-section of a flexible handle 500 is shown. The flexible handle 500 may be similar to the handle 120 and/or 300 previously described in that it couples the needle 114 to the cryogen line 110 and permits relative movement between the needle 114 and the cryogen line 110. The flexible handle 500 may permit movement and positioning of the needle 114 relative to the cryogen line 110 as previously described.

In this example, the handle 500 includes an outer member 502, a deformable member 504, and a cryogen supply tube 506, centered around center axis 508. The outer member 502, the deformable member 504, and the cryogen tube 506 may be similar to the comparable elements of handle 300. In this example, the handle 500 includes a limiting member 516 positioned in the annular space 510. The limiting member 516 is positioned radially inward of the outer member 502 and radially outward of the deformable member 504. The deformable inner member 504 and the cryogen supply tube 506 define a cryogen flow path 514 in which a cryogen flows toward a tip 202 of the needle 114 through the cryogen supply tube 506 and away from the tip of the needle in a space 512 between an outer surface of the cryogen supply tube 506 and an inner surface of the deformable inner member 504.

The limiting member 516 may be configured to limit or restrict the amount of bending that can be performed with the handle 500. The limiting member 516 may be configured to reduce the likelihood that one or more members of the handle 500 may kink and/or rupture during use. In some examples, the limiting member 516 may limit an angle that the handle can be flexed. The limiting member 516 may limit the angle of flex of the handle 500 to about 90 degrees in some examples. In other examples, the limiting member 516 may restrict the bending of the handle 500 to other amounts or ranges.

In this example, the limiting member 516 may have a helical configuration. In other examples, the limiting member 516 may have a coil spring configuration. In other examples, a corrugated or oscillating outer surface may be used. In the example shown, the limiting member is a separate member and positioned in a space (510) between concentric members of the handle 500. In other examples, the limiting member 516 may integrally formed and/or embedded into one of the outer member 502 and/or the deformable member 504.

Referring now to FIG. 6, another example cross-section of a flexible handle 600 is shown. The flexible handle 600 may be similar to the handle 120 and/or 300 previously described in that it couples the needle 114 to the cryogen line 110 and permits relative movement between the needle 114 and the cryogen line 110. The flexible handle 600 may permit movement and positioning of the needle 114 relative to the cryogen line 110 as previously described.

In this example, the handle 600 includes an outer member 602, a deformable member 604, and a cryogen supply tube 606. The outer member 602, the deformable member 604, and the cryogen tube 606 may be similar to the comparable elements of handle 300. The outer member 602, the deformable member 604, and the cryogen tube 606 may be concentrically positioned around a center axis 608 and form annular spaces 610 and 612. The lumen 614 of the cryogen tube 606 may supply cryogen to the needle of the cryoprobe.

In this example, the outer member 602 may include a configuration with reinforcements 616. In this example, reinforcements 616 are projections that extend outward from an outer surface of the outer member 602. The reinforcements 616 may be longitudinally formed in the outer member 602 and extend along the length of the outer member 602. The reinforcements 616 may be integrally formed into the wall of the outer member 602. In other examples, the reinforcements may be separate pieces joined to the outer member or extruded with or into the material of the outer member 602.

The reinforcements 616 may increase a strength of the outer member in a particular plane or in a particular direction relative to the center axis 608. The reinforcements 616 may restrict or limit a bending or movement of the outer member 602 and thus, the handle 600 in a particular plane or direction. In this example, the outer member 602 includes two reinforcements 616 positioned with one positioned on each side of the outer member 602 opposite to one another. In other examples, other quantities or other orientations or positioning of the reinforcements 616 may be used. The reinforcements 616 have an outer rectangular shape in this example, but other shapes such as triangular, circular, others may be used.

The flexible handles of the present disclosure may be used to perform more effective ablation treatments. The flexible handles allow for positioning of cryogen lines at desired positions to minimize interference with other treatment apparatuses and device as well as with other cryoprobes. The flexible handles also minimize and/or reduce a likelihood that a cryoprobe needle will be moved or shift after it has been positioned at a desired location relative to a target tissue.

Various methods of use are contemplated using the flexible handles and cryoprobes of the present disclosure. In one example method, the method may include positioning a needle of a cryoprobe at a desired position relative to a target tissue in a patient. The cryoprobe may be configured with the handle in a desired orientation while the needle is positioned at or near the target tissue.

At the conclusion of the positioning of the cryoprobe needle, the handle of the cryoprobe may be flexed or bent to desired position. Such bending may be made in order to move the cryogen line of the cryoprobe away from another piece of medical equipment. The bending may be made so reduce a height of the cryoprobe so that it does not extend an excessive distance above the patient so that the patient can be easily moved into or out of a piece of imaging equipment. Still further the bending may be made to reduce the height and thus reduce the moment arm of the cryoprobe so that the needle remains in the desired location during treatment. Still further the bending may be made to position the cryoprobe and/or the cryogen line in a desired position to increase the likelihood of effective treatment.

The cryogen may then be initiated to the cryoprobe with the cryoprobe in the newly bent position. The cryoablation treatment may be formed in this configuration. Such flexibility is not available in existing cryoprobe and is a significant improvement over the rigid, non-flexible cryoprobes currently available.

The following is a list of non-limiting illustrative embodiments disclosed herein:
Illustrative embodiment 1: A cryoprobe comprising: a needle; and a handle configured to fluidly couple the needle to a cryogen line, the handle configured to flex to change a position of the needle relative to the cryogen line.
Illustrative embodiment 2. The cryoprobe of illustrative embodiment 1, wherein the handle is configured to flex from a first position in which a longitudinal axis of the needle is aligned with a longitudinal axis of the cryogen supply line to a second position in which the longitudinal axis of the needle is positioned at an angle relative to the longitudinal axis of the cryogen supply line.
Illustrative embodiment 3. The cryoprobe of illustrative embodiment 2, wherein the angle of the second position of the cryoprobe is in a range between 180 degrees and 90 degrees.
Illustrative embodiment 4. The cryoprobe of any of illustrative embodiments 1 to 3, wherein the handle comprises a deformable member configured to maintain a plurality of positions of the needle relative to the cryogen line.
Illustrative embodiment 5. The cryoprobe of any of illustrative embodiments 1 to 4, wherein the handle comprises: a flexible outer member defining an inner cavity; and a deformable inner member positioned in the inner cavity, the deformable inner member configured to maintain a position of the needle relative to the cryogen line.
Illustrative embodiment 6. The cryoprobe of illustrative embodiment 5, wherein the flexible outer member and the deformable inner member each have a cylindrical shape.
Illustrative embodiment 7. The cryoprobe of any of illustrative embodiments 5 or 6, wherein the handle further comprises a cryogen supply tube positioned radially inward of the deformable inner member in the inner cavity.
Illustrative embodiment 8. The cryoprobe of illustrative embodiment 7, wherein the deformable inner member and the cryogen supply tube define a cryogen flow path in which a cryogen flows toward a tip of the needle through the cryogen supply tube and away from the tip of the needle in a space between an outer surface of the cryogen supply tube and an inner surface of the deformable inner member.
Illustrative embodiment 9. The cryoprobe of any of illustrative embodiments 5 to 8, wherein the handle further comprises a limiting member positioned in the inner cavity radially outward of the deformable inner member, the limiting member configured to limit bending of the handle.
Illustrative embodiment 10. The cryoprobe of any of illustrative embodiments 1 to 9, wherein the handle comprises a cryogen supply tube and an outer flexible member, the outer flexible member comprising at least one embedded wire configured to maintain a position of the needle relative to the cryogen line.
Illustrative embodiment 11. The cryoprobe of illustrative embodiment 10, wherein the at least one embedded wire comprises a plurality of embedded wires.
Illustrative embodiment 12.The cryoprobe of any of illustrative embodiments 10 or 11, wherein each wire of the plurality of embedded wires are equally spaced around a circumference of the outer flexible member.
Illustrative embodiment 13. The cryoprobe of any of illustrative embodiments 1 to 3, wherein the handle comprises: a flexible outer member; and a deformable member configured to maintain a position of the needle relative to the cryogen line.
Illustrative embodiment 14. The cryoprobe of any of illustrative embodiments 5 to 13, wherein the flexible outer member comprises at least one longitudinal reinforcement configured to limit bending of the flexible outer member in at least one plane.
Illustrative embodiment 15. The cryoprobe of illustrative embodiment 14, wherein the at least one longitudinal reinforcement comprises a first reinforcement and a second reinforcement positioned on opposite sides of the flexible outer member, the first reinforcement and the second reinforcement comprising enlarged portions of a wall of the flexible outer member.
Illustrative embodiment 16. The cryoprobe of any of illustrative embodiments 1 to 15, wherein the handle is configured to flex in a plurality of planes relative to an axis of the cryogen line.
Illustrative embodiment 17. The cryoprobe of any of illustrative embodiments 1 to 16, wherein the handle is configured to flex in at least two orthogonal planes relative to an axis of the cryogen line.
Illustrative embodiment 18. The cryoprobe of any of the foregoing illustrative embodiments, wherein the handle comprises a deformable member configured to permanently deform to maintain a position of the needle relative to the cryogen line.
Illustrative embodiment 19. A cryoablation system comprising: the cryoprobe of any of the foregoing illustrative embodiments; and a cryoablation delivery apparatus configured to deliver a cryogen to the cryoprobe.
Illustrative embodiment 20. A method of using a cryoprobe comprising: positioning the cryoprobe of any of the foregoing illustrative embodiments at a target tissue; bending a handle of the cryoprobe from a first position to a second position to orient the cryogen line at a desired position relative to the needle; and initiating a flow of cryogen to the cryoprobe while the handle is in the second position.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

## Claims

1. A cryoprobe (112) comprising:
a needle (114); and
a handle (116, 120, 300, 400, 500, 600) configured to fluidly couple the needle to a cryogen line (110), the handle (116, 120, 300, 400, 500, 600) configured to flex to change a position of the needle (114) relative to the cryogen line (110).

2. The cryoprobe of claim 1, wherein the handle (116,120, 300, 400, 500, 600) is configured to flex from a first position in which a longitudinal axis of the needle (212) is aligned with a longitudinal axis of the cryogen supply line (214) to a second position in which the longitudinal axis of the needle (212) is positioned at an angle relative to the longitudinal axis of the cryogen supply line (214).

3. The cryoprobe of claim 2, wherein the angle of the second position of the cryoprobe is in a range between 180 degrees and 90 degrees.

4. The cryoprobe of claim 1, 2 or 3, wherein the handle (116, 120, 300, 400, 500, 600) comprises a deformable member (208, 304, 404, 504, 604) configured to maintain a plurality of positions of the needle (114) relative to the cryogen line (110).

5. The cryoprobe of any one of claims 1 to 4, wherein the handle (120, 300, 500, 600) comprises:
a flexible outer member (206, 302, 502, 602) defining an inner cavity; and
a deformable inner member (208, 304, 504, 604) positioned in the inner cavity, the deformable inner member configured to maintain a position of the needle (114) relative to the cryogen line (110), wherein, optionally, the flexible outer member (206, 302, 502, 602) and the deformable inner member (208, 304,504, 604) each have a cylindrical shape.

6. The cryoprobe of claim 5, wherein the handle (120, 300, 500, 600) further comprises a cryogen supply tube (210, 306, 506, 606) positioned radially inward of the deformable inner member (208, 304, 504, 604) in the inner cavity.

7. The cryoprobe of claim 6, wherein the deformable inner member (208, 304, 504, 604) and the cryogen supply tube (210, 306, 506, 606) define a cryogen flow path (514, 614) in which a cryogen flows toward a tip (202) of the needle (114) through the cryogen supply tube (210, 306, 506, 606) and away from the tip of the needle in a space (512) between an outer surface of the cryogen supply tube (210, 306, 506, 606) and an inner surface of the deformable inner member (208, 304, 504, 604).

8. The cryoprobe of claim 5, wherein the handle (500) further comprises a limiting member (516) positioned in the inner cavity radially outward of the deformable inner member, the limiting member configured to limit bending of the handle.

9. The cryoprobe of any one of claims 1 to 4, wherein the handle (400) comprises a cryogen supply tube (406) and an outer flexible member (402), the outer flexible member comprising at least one embedded wire (404) configured to maintain a position of the needle (114) relative to the cryogen line (110) wherein, optionally, the at least one embedded wire (404) comprises a plurality of embedded wires (404) and, optionally, each wire of the plurality of embedded wires (404) are equally spaced around a circumference of the outer flexible member (402).

10. The cryoprobe (112) of any one of the preceding claims, wherein the handle (116, 120, 300, 400, 500, 600) comprises:
a flexible outer member (206, 302, 402, 502, 602); and
a deformable member (208, 304, 404, 504, 604) configured to maintain a position of the needle (114) relative to the cryogen line (110).

11. The cryoprobe of claim 10, wherein the flexible outer member (602) comprises at least one longitudinal reinforcement (616) configured to limit bending of the flexible outer member in at least one plane wherein, optionally, the at least one longitudinal reinforcement (616) comprises a first reinforcement and a second reinforcement positioned on opposite sides of the flexible outer member (602), the first reinforcement and the second reinforcement comprising enlarged portions of a wall of the flexible outer member (602).

12. The cryoprobe of any one or the preceding claims, wherein the handle (116, 120, 300, 400, 500, 600) is configured to flex in a plurality of planes relative to an axis of the cryogen line (110).

13. The cryoprobe of any one of the preceding claims, wherein the handle (116, 120, 300, 400, 500, 600) is configured to flex in at least two orthogonal planes relative to an axis of the cryogen line (110).

14. The cryoprobe of any one of the preceding claims, wherein the handle (116, 120, 300, 400, 500, 600) comprises a deformable member configured to permanently deform to maintain a position of the needle relative to the cryogen line.

15. A cryoablation system (100) comprising:
the cryoprobe of any one of the preceding claims; and
a cryoablation delivery apparatus (106) configured to deliver a cryogen to the cryoprobe (112).
